# EUROPÄISCHE PATENTSCHRIFT

(11) **EP 3 436 183 B1**
(45) Veröffentlichungstag und Bekanntmachung des Hinweises auf die Patenterteilung: **03.06.2020**
(21) Anmeldenummer: 17719153.3
(22) Anmeldetag: 29.03.2017
(51) Int. Cl.: B01D 63/02, A61M 1/16

(54) **HOHLFASERMEMBRANVORRICHTUNG FÜR DEN STOFFAUSTAUSCH UND VERFAHREN ZUR HERSTELLUNG**
HOLLOW-FIBRE MEMBRANE FOR MASS TRANSFER, AND METHOD OF PRODUCTION
DISPOSITIF D'ÉCHANGE DE SUBSTANCE ET PROCÉDÉ DE FABRICATION

(30) Priorität: 29.03.2016 DE 102016003611
(43) Veröffentlichungstag der Anmeldung: 06.02.2019
(73) Patentinhaber: enmodes GmbH, 52070 Aachen (DE)
(72) Erfinder: BORCHARDT, Ralf, 52066 Aachen (DE)
(74) Vertreter: Cohausz Hannig Borkowski Wißgott
(86) Internationale Anmeldenummer: PCT/EP2017/000377
(87) Internationale Veröffentlichungsnummer: WO 2017/167443

(56) Entgegenhaltungen:
- EP-A1- 0 285 812
- EP-A1- 0 862 943
- EP-A1- 1 674 149
- EP-A1- 2 042 228
- EP-A1- 2 153 882
- EP-A2- 0 329 980
- DE-A1- 2 825 065
- JP-A- H05 228 345
- JP-A- 2007 216 175
- JP-U- S63 168 004
- US-A1- 2015 197 431

## Beschreibung

Die Erfindung betrifft ein auf einen Kern gewickeltes Hohlfaserpaket aus einer Vielzahl stoffpermeabler Hohlfasern von wenigstens einer Hohlfasermatte, in welcher die Hohlfasern mit Kettfäden auf Abstand gehalten sind. Die Erfindung betrifft auch eine Vorrichtung für den Stoffaustausch zwischen Blut und einem Gas/Gasgemisch, umfassend eine blutdurchströmbare Kammer, in der eine Vielzahl stoffpermeabler Hohlfasern als gewickeltes oder gefaltetes Hohlfaserpaket angeordnet ist, wobei die Hohlfasern von einem Austauschmedium, insbesondere einem Gas / Gasgemisch durchströmbar und von dem Blut umströmbar sind.

Die Erfindung betrifft auch ein Verfahren zur Herstellung eines Hohlfaserpaketes einer solchen Vorrichtung.

Vorrichtungen dieser Art sind im Stand der Technik bekannt und werden z.B. als Oxygenatoren eingesetzt. Solche Vorrichtungen können jedoch auch zum Zweck der Reinigung des Blutes von belastenden Stoffen eingesetzt werden, beispielsweise bei der Dialyse.

Bei Oxygenatoren soll mit solchen Vorrichtungen das durch die Vorrichtung hindurchfließende Blut mit Sauerstoff angereicht und von Kohlendioxid abgereichert werden. Dieser Prozeß erfolgt dadurch, dass durch das Gas / Gasgemisch als Austauschmedium, welches durch die stoffpermeablen Hohlfasern fließt ein Konzentrationsgefälle der auszutauschenden Stoffe (O₂ und CO₂) zwischen dem Blut und dem Gas / Gasgemisch erzeugt wird, so dass durch den Vorgang der Permeation Sauerstoff aus dem Gas / Gasgemisch durch die Hohlfaserwände in das Blut übertritt und Kohlendioxid aus dem Blut durch die Hohlfaserwände in das Gas / Gasgemisch übertritt. Solche Oxygenatoren können z.B. in Herz-Lungenmaschinen zum Einsatz kommen.

Je nach Anwendung werden geeignete Austauschmedien eingesetzt um die Reinigung des Blutes von einem belastenden Stoff und ggfs. die Anreicherung mit einem anderen gewünschten Stoff durch Permeation zu erzielen.

Typische Vorrichtungen dieser Art werden bislang so hergestellt, dass wenigstens eine Matte mit einer Vielzahl hintereinander liegender äquidistant beabstandeter stoffpermeabler Hohlfasern in mehreren Lagen aufgewickelt, insbesondere auf einem Kern aufgewickelt wird, durch den später auch der Blutzu- oder -abfluss erfolgen kann oder aber wenigstens eine solche Matte mehrfach lagenbildend gefaltet wird. In einer solchen Matte liegen die Hohlfasern bevorzugt alle parallel zueinander, wobei sich die Enden der jeweiligen Hohlfasern auf gegenüberliegenden Seiten der Matte befinden. Das Falten oder Wickeln kann z.B. jeweils um eine Linie parallel zu den Längserstreckungsrichtungen der Fasermatten erfolgen.

Die Fasern können auch so orientiert sein, dass Fasern benachbarter Lagen des gewickelten oder gefalteten Paketes in einem Winkel ungleich 0° bzw. ungleich 180° liegen. Z.B. können auch zwei oder mehr aufeinanderliegende Matten gewickelt oder gefaltet werden, wobei die Fasern verschiedener Matten zueinander einen Winkel ungleich 0° bzw. ungleich 180° aufweisen.

In einer jeweiligen Matte können die Faser auch in einem Winkel ungleich 90 Grad zu der Erstreckungsrichtung der haltenden Kettfäden angeordnet sein.

Die vorgenannten Verfahren der Herstellung können auch bei der erfindungsgemäßen Vorrichtung jeweils bevorzugt eingesetzt werden, insbesondere hierbei erfindungsgemäß weitergebildet werden.

Durch solche Herstellungsarten kann sich ein Hohlfaserpaket ergeben, mit zumindest im Wesentlichen in einer axialen Richtung erstreckten Hohlfasern, das in einem Gehäuse untergebracht werden kann. Im Querschnitt betrachtet (senkrecht zur Fasererstreckung oder senkrecht zur Paketerstreckung) ist die Hohlfaserdichte des Hohlfaserpaketes aufgrund der Äquidistanz der Hohlfasern in der Matte im Wesentlichen überall gleich.

Durch den in Fachkreisen bekannten Vorgang des Verpottens werden die axialen Enden der Hohlfasern zueinander und gegenüber einem Gehäuse mit einer Verpottungsmasse abgedichtet, insbesondere in dafür eingesetzten Zentrifugen oder Vergussanlagen.

Über die freibleibenden oder nach dem Verpotten von Verpottungsmasse befreiten axialen Enden der Hohlfasern kann sodann das Austauschmedium, z.B. ein Gas / Gasgemisch durch die Hohlfasern geleitet werden und durch im Gehäuse angeordnete Zu- und Abführungen kann Blut zwischen die Hohlfasern im Bereich zwischen den axial endseitigen Verpottungsstellen geführt werden. Durch die Gehäusewände und die Verpottungsstellen wird eine Kammer ausgebildet, in welcher die Hohlfasern angeordnet sind und die durch das Blut durchflossen werden kann, insbesondere ohne dass das Blut direkten Kontakt zum Austauschmedium, insbesondere dem Gas / Gasgemisch hat.

Typische zylindrische Vorrichtungen dieser Art haben einen Blutzufluss oder - abfluss durch einen hohlen Kern, auf den die wenigstens eine Matte gewickelt ist. Der Kern, der einen hohlen Kanal bildet ist im Bereich eines axialen Endes zum Kammerinneren hin offen, insbesondere in radialer Richtung nach außen offen, so dass Blut dort zwischen dem Kern und dem Kammerinneren übertreten kann. Am gegenüberliegenden axialen Ende erfolgt, bevorzugt radial außenliegend, ebenso ein Übergang für das Blut zwischen dem Kammerinneren und einem bevorzugt in Umfangsrichtung angeordneten Raum. Welcher der vorgenannten jeweiligen Übergänge zur Kammer als Zufluß und als Abfluß für das Blut genutzt wird, ist im Wesentlichen unerheblich.

Die Erfindung betrifft bevorzugt die vorgenannte Konstruktion von Vorrichtungen, bevorzugt mit einem radial außen liegenden Blutabfluss an einem der axialen Enden und einem radial innenliegenden über den Wickelkern gebildeten Blutzufluss am anderen der axialen Enden. Die Erfindung ist jedoch nicht hierauf beschränkt.

Innerhalb der Kammer erfolgt allgemein der Blutfluß im Wesentlichen auf direktem Weg zwischen Blutzufluß und - abfluß durch die Kammer hindurch. Vom direkten Weg abweichende Bereiche sind geringer durchströmt, so dass über den Querschnitt eines Hohlfaserpaketes senkrecht zur Axialrichtung betrachtet eine ungleichmäßige Strömung vorherrscht. Dies ist jedoch nachteilig und führt in diesen geringer durchströmten Bereichen zu schlechterem Stoffaustausch und ggfs. auch zu Thrombenbildung.

Bei der vorgenannten Konstruktion der Vorrichtung ist der Blutfluß somit im Wesentlichen zwischen den axialen Endbereichen von radial innenliegend schräg zur axialen Richtung nach radial außenliegend. Es ergibt sich somit über den Querschnitt eines Hohlfaserpaketes senkrecht zur Axialrichtung betrachtet eine ungleichmäßige Strömung. Die maximale Strömung liegt hier in einem Ringbereich, der sich in axialer Richtung betrachtet von radial innenliegend nach radial außenliegend verlagert.

Besonders schlecht durchströmt sind allgemein diejenigen Stellen, die beim Gehäuse an der jeweiligen axialen Position dem Einlass oder dem Auslass von Blut gegenüberliegen. Bezogen auf die genannte spezielle Konstruktion erfolgt eine schlechtere Durchströmung daher bei demjenigen axialen Ende, wo der Übergang zur Kammer radial innen am hohlen Kern liegt somit an den radial außenliegenden Umfangsbereichen der Kammer und bei demjenigen axialen Ende, wo der Übergang zur Kammer radial außen liegt dementsprechend an dem radial innenliegenden Umfangsbereich, der den an diesem axialen Ende geschlossenen Kern umgibt.

Die Publikation DE 28 25 065 A1 zeigt ein Hohlfaserpaket, bei dem die Hohlfasern um einen am Ende abgestuft ausgebildeten Kern gewickelt sind.

Die Publikation JP 2007 216175 A zeigt einen im axialen Ende des Hohlfaserpaketes angeordneten kegelförmigen Körper, was zu einer axialen Änderung der Dichte der Hohlfaser führt.

Die Publikation EP 1 674 149 A1 beschreibt es, Formkörper endseitig in ein Bündel von Membranen einzuschieben und hierdurch eine Spreizung des Bündels zu erzielen.

Bei der Publikation EP 0 285 812 A1 wird eine Änderung der Hohlfaserdichten durch Querschnitt- oder Anordnungsunterschiede in den Hohlfasermatten erzeugt.

Die Publikation EP 2 042 228 A1 offenbart die Verwendung von Einsätzen in Hohlfaserbündeln, die nach dem Verpotten wieder entfernt werden.

Es ist somit eine Aufgabe der Erfindung die Strömung des Blutes in der Kammer über den Querschnitt (senkrecht zur Axialerstreckung) des Hohlfaserpaketes hinweg zu vergleichmäßigen, d.h. in den nach bisheriger Ausgestaltung benachteiligten Strömungsbereichen die Strömung zu vergrößern.

Diese Aufgabe wird dadurch gelöst, dass im Hohlfaserpaket betrachtet im Querschnitt senkrecht zur Hohlfasererstreckung oder senkrecht zur Paketerstreckung, die Dichte an Hohlfasern lokal unterschiedlich ist, wobei ein Bereich geringerer Faserdichte erzeugt ist durch wenigstens einen in das Hohlfaserpaket, zumindest axial endseitig eingesetzten Platzhalter, der zwischen zwei Lagen von durch Kettfäden verbundenen Hohlfasern eingewickelt ist.

Im Verfahren wird die Aufgabe dadurch gelöst, dass durch Beeinflussung des Abstandes der Hohlfasern zueinander vor oder während des Wickelns im Querschnitt senkrecht zur Hohlfasererstreckung Bereiche mit unterschiedlicher Dichte an Hohlfasern erzeugt werden, wobei die Beeinflussung des Abstandes der Hohlfasern zueinander erfolgt durch Einwickeln von wenigstens einem Platzhalter zwischen zwei Lagen wenigstens einer Hohlfasermatte.

Durch eine solche im Querschnitt lokal unterschiedliche Ausbildung der Hohlfaserdichte kann der Strömungswiderstand bewußt dort reduziert werden, wo die Hohlfaserdichte reduziert wird, z.B. durch Vergrößerung des Abstandes zwischen benachbarten Hohlfasern des Paketes. Bislang im Stand der Technik strömungstechnisch benachteiligte Bereiche können so erfindungsgemäß mit einer stärkeren Strömung versehen werden, indem bewußt dort die Hohlfaserdichte reduziert wird.

Es kann hierfür bevorzugt wenigstens ein Bereich geringerer Faserdichte im Vergleich zur Faserdichte umgebender Bereiche im Hohlfaserpaket angeordnet sein. Bevorzugt werden hierbei Bereiche verglichen, die im Querschnitt senkrecht zur axialen Erstreckung jeweils vollständig im Faserbündel liegen, insbesondere also keine Bereiche, die sich über den Randbereich des Faserbündels hinaus erstrecken.

Wenigstens ein solcher Bereich reduzierter Faserdichte kann z.B. beabstandet, bevorzugt gegenüberliegend, insbesondere radial gegenüberliegend angeordnet sein zu einem Bluteinlassbereich und/oder Blutauslaßbereich der Kammer.

Die Anordnung kann so ausgebildet sein, dass im Querschnitt betrachtet ein Dichtegradient gebildet wird, der vom Bereich reduzierter Faserdichte in Richtung zum Bluteinlass und/oder Blutauslass zeigt, d.h. in einer axial endseitigen Querschnittsebene nimmt bevorzugt die Dichte in Richtung zum Einlass bzw. Auslass zu. Bezogen auf die genannte bevorzugte Konstruktion erfolgt somit zumindest axial endseitig eine Dichtzunahme in einer radialen Richtung.

Die Anordnung der dichtereduzierten Bereiche zu Bereichen höherer Faserdichte kann über die axiale Erstreckung eines Hohlfaserpaketes gleich bleiben, d.h. in allen axialen Querschnittsebenen kann das Dichteprofil gleich sein. Bevorzugt und besonders bei der genannten bevorzugten Konstruktion ist das Dichteprofil jedoch in verschiedenen axialen Querschnittsebenen unterschiedlich. Insbesondere kann sich mit zunehmender Axialposition ein Bereich, bevorzugt Ringbereich reduzierter Dichte von radial außenliegend nach radial innenliegend verschieben. D.h. dass z.B. in einer axialen Mittenposition ein Ring reduzierter Faserdichte radial zwischen, insbesondere mittig zwischen Ringbereichen höherer Faserdichte liegt.

Erfindungsgemäß kann ein Bereich geringerer Faserdichte eine solche Faserdichte aufweisen, die mindestens 5 %, bevorzugt mindestens 10%, noch weiter bevorzugt mindestens 15% kleiner ist als die Faserdichte in einem umgebenden Bereich des Hohlfaserpaketes oder als die höchste Faserdichte im Hohlfaserpaket.

Erfindungsgemäß kann in einem Minderanteil der Querschnittsfläche des Hohlfaserpaketes die Faserdichte reduziert sein, insbesondere mindestens um das vorgenannte Maß gegenüber einem Majoritätsanteil der Querschnittsfläche, in welchem die Faserdichte konstant größer ist oder zumindest innerhalb eines Intervalls größer ist. Ein solches Intervall weist bevorzugt Intervallgrenzen auf, deren Werte um nicht mehr als 5% voneinander abweichen.

Die Erfindung sieht vor, die Beeinflussung des Abstandes der Hohlfasern im fertigen Hohlfaserpaket vorzunehmen durch Einwickeln von wenigstens einem Platzhalter zwischen zwei Lagen wenigstens einer Hohlfasermatte. Ein solcher Platzhalter kann also z.B. eingewickelt oder eingefaltet werden zwischen zwei Lagen ein und derselben Matte oder auch zwischen zwei Lagen, die von zwei verschiedenen Matten gebildet werden.

Durch einen solchen Platzhalter wird somit am Ort des Platzhalters der Abstand der Lagen vergrößert gegenüber Bereichen vor und hinter dem Platzhalter. Am Ort eines Platzhalters berühren die Lagen bzw. deren Hohlfasern bevorzugt den Platzhalter. Bevorzugt sind dabei bei einem im Querschnitt kreisförmigen Platzhalter die beiden Lagen in der Umfangsrichtung jeweils um weniger als 180 Grad herumgelegt, insbesondere in einer angenommenen seitlichen Schnittansicht ist eine Lage oben und eine unten um den Platzhalter herumgelegt.

An sich senkrecht zur axialen Richtung der Hohlfasern / eines Platzhalters gegenüberliegenden Bereichen sind die zwei Lagen jeweils wieder zusammengeführt, insbesondere abstandslos zusammengeführt, insbesondere so, dass sich die Lagen bzw. die Hohlfasern der Lagen nach dem Wieder-Zusammenlegen berühren. An den benannten gegenüberliegenden Bereichen gehen die beiden Lagen somit von einem jeweils den Platzhalter kontaktierenden Zustand in einen sich gegenseitig kontaktierenden Zustand über. Zwischen diesen Bereichen ist ein Freiraum umschlossen, der die Dichtereduktion bewirkt, insbesondere der also in der Verbindungsrichtung der beiden genannten gegenüberliegenden Bereiche zwischen der Platzhalteroberfläche und dem Punkt erstmaliger Wieder-Zusammenführung der Lagen liegt.

Sofern sich der Platzhalter über die gesamte axiale Länge der Hohlfasern erstreckt erfolgt wiederum eine Beeinflussung der Faserdichte über die gesamte axiale Länge.

Es kann aber auch sein, dass ein Platzhalter oder mehrere nur im Bereich der Faserenden zwischen zwei Lagen einer gewickelten Matte angeordnet wird. So kann insbesondere nur im Bereich der axial endseitig angeordneten Bluteinlass- bzw. Auslassbereiche eine Änderung der Faserdichte in Richtung reduzierter Dichte erfolgen.

Es kann auch sein, dass ein Platzhalter nur in dem Bereich, insbesondere dem Verpottungsbereich, zwischen den axialen Enden der Fasern zwischen zwei Lagen angeordnet ist, sich ein Platzhalter als nicht bis in den Endbereich der Fasern erstreckt.

Allgemein kann es vorgesehen sein, Platzhalter mit einer Länge kleiner als die axiale Länge der Hohlfasern in Bereichen an oder zwischen den axialen Enden der Hohlfasern beim Wickeln wenigstens einer Matte zwischen zwei Lagen anzuordnen. So kann an grundsätzlich beliebigen Stellen im Hohlfaserpaket eine Dichtereduktion erfolgen. Insbesondere erfolgt die Dichtereduktion dabei in axialer Richtung im Wesentlichen über die Länge, die durch den Platzhalter gegeben ist.

Es kann hier vorgesehen sein, dass solche Platzhalter im Hohlfaserpaket verbleiben. Platzhalter, die axial endseitig zugänglich sind können auch vor dem Verpotten der Hohlfasern aus dem gebildeten Paket herausgezogen werden, wobei die Hohlfasern im Wesentlichen ihre zuvor eingenommene Lage zu einander beibehalten. Hierdurch wird sogar der Raum insgesamt frei, der zuvor durch den Platzhalter eingenommen wurde und trägt zur lokalen Hohlfaserdichtereduktion bei. Sofern ein im Querschnitt kreisförmiger Platzhalter aus dem Paket herausgezogen wird verbleibt ein Freiraum im Paket, der zuvor vom Platzhalter eingenommen wurde. Dieser Freiraum wird von den beiden Lagen der wenigstens einen Hohlfasermatte teilkreisförmig umgeben, da diese Lagen zuvor an der Mantelfläche des Platzhalters angelegen haben. Allgemein, insbesondere sofern der Platzhalter nicht kreisförmig war, zeichnen die beiden Lagen in ihrem Verlauf ebenso die Form der Mantelfläche des entfernten Platzhalters nach.

Im Hohlfaserpaket verbleibende Platzhalter können so ausgebildet sein, dass sie von Blut durchströmbar sind, z.B. dadurch, dass sie aus Hohlprofilen, z.B. Rohren gebildet sind, insbesondere deren Mantelfläche perforiert ist. Bei einer Perforation der Mantelfläche kann die Perforation so gebildet sein, dass diese eine Filterfläche bildet.

Durch Ausbildung des wenigstens einen Platzhalters als Hohlprofil, insbesondere als Rohr, bevorzugt nicht perforiertes Rohr wird auch die Möglichkeit erschlossen, dass ein solcher Platzhalter von einem Temperierungsmedium durchströmt werden kann. So können solche Platzhalter als Wärmetauscher dienen, insbesondere um das Blut im Betrieb zu temperieren. Um dies zu realisieren, können solche hohlen Platzhalter an ihren jeweiligen Enden an einen eigenen Fuidkreislauf angeschlossen sein.

Neben der Möglichkeit, einen Platzhalter hohl auszubilden, kann die Erfindung auch vorsehen, einen Platzhalten mit massivem Querschnitt auszugestalten. Bevorzugt kann ein solcher Platzhalter außer seiner Platzhalterfunktion somit keine weitere Funktion im Bündel übernehmen.

Ein Platzhalter, insbesondere ein im Paket verbleibender, kann bevorzugt einen Querschnitt aufweisen, der größer ist als der Querschnitt einer Hohlfaser. Bevorzugt kann der Querschnitt, insbesondere Durchmesser mindestens 2-mal, weiter bevorzugt mindestens 3-mal so groß sein, wie der Querschnitt einer jeweiligen Hohlfaser.

Hierdurch ergeben sind besonders an den gegenüberliegenden äußeren Bereichen des Platzhalters, in welchen diejenigen Lagen der Matte aneinander angrenzen zwischen denen der Platzhalter angeordnet ist, Freiräume zwischen Platzhalter und Hohlfasern, die zur Dichtereduktion führen.

Die Erfindung kann in einer ebenso möglichen Ausführung auch vorsehen, dass ein Platzhalter durch eine stoff-permeable Hohlfaser gebildet ist, die am Stoffaustausch teilnimmt, insbesondere genauso wie die anderen Hohlfasern. Eine solche Platzhalter-Hohlfaser ist somit hohl ausgebildet, vom Austauschmedium (insbesondere Gas) durchflossen, insbesondere also endseitig ebenso verpottet, und im Querschnitt, bevorzugt Durchmesser größer, insbesondere wenigstens 2-mal, bevorzugt wenigstens 3-mal größer als die jeweiligen Hohlfasern der wenigstens einen Matte. Eine solche Hohlfaser kann separat zu den Hohlfasern der Matte sein.

Allgemein können die Hohlfasern einer Matte, abgesehen, von der nachfolgende benannten Ausführung alle denselben Querschnitt aufweisen.

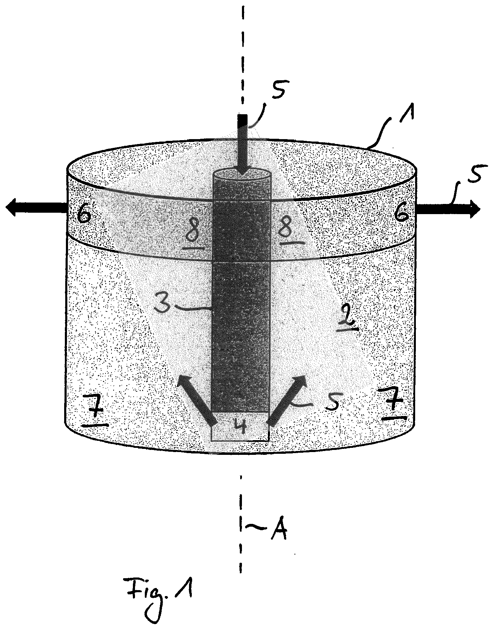

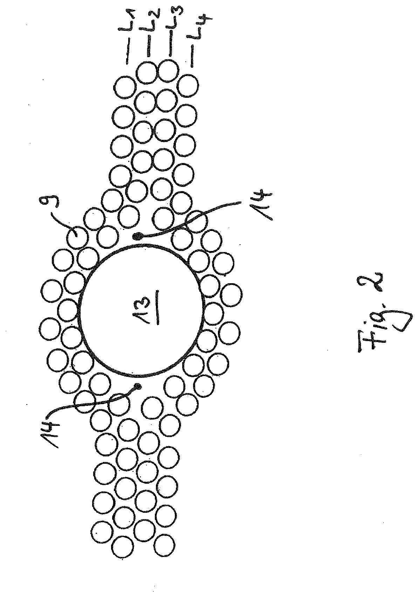

Ein Ausführungsbeispiel wird nachfolgend anhand der Figuren 1 und 2 erläutert.

Figur 1 zeigt eine bevorzugte Konstruktion einer Vorrichtung der Erfindung. Das Gehäuse 1 bildet einen Zylinder mit Kreisquerschnitt mit einer inneren Kammer 2 in der parallel zur Achse A nicht visualisierte stoffpermeable Hohlfasern angeordnet sind. Axial endseitig werden diese durch ein Austauschmedium beaufschlagt, dass durch die Hohlfasern strömt, z.B. ein Gas oder Gasgemisch.

Die Hohlfasern sind um den hohlen kanalbildenden Kern 3 angeordnet, dadurch, dass wenigstens eine Matte aus solchen Hohlfasern um diesen herumgewickelt wurde. Der Kern 3 weist am hier unteren Ende eine Öffnung 4 auf, die somit bezogen auf das Gehäuse 1 radial innen liegt. Das obere Ende des Kerns 3 bildet hier den Bluteinlass.

In Richtung der Pfeile 5 strömt das am oberen Ende des Kerns eingelassene Blut zunächst durch den Kern nach unten, aus der Öffnung 4 in die Kammer 2 und dort zwischen den Hohlfasern in Richtung zum Blutauslaß 6, der im Wesentlichen in Umfangsrichtung ausgebildet ist und radial außen liegt bezogen auf das Gehäuse 1.

Bei zumindest im Wesentlichen konstanter Hohlfaserdichte (betrachtet in einem Querschnitt senkrecht zur Achse A) verläuft die Strömung hauptsächlich von unten innen nach oben außen. Im unteren Bereich sind somit die radial außenliegenden, also der für das Blut als Einlass in die Kammer dienenden Öffnung 4 radial gegenüberliegenden Bereiche 7 schlechter durchströmt. Am oberen axialen Ende trifft das für die radial innen liegenden Bereiche 8 zu.

Die Erfindung trägt dafür Sorge, dass durch lokale Reduzierung der Hohlfaserdichte in diesen dem Einlass und Auslass gegenüberliegenden Bereichen 7 und 8 die Strömungswiderstrände verkleinert werden gegenüber dem Fall einer über den Querschnitt gleichbleibenden Faserdichte. Hierdurch nimmt die Strömung auch in den ansonsten benachteiligten Bereichen 7 und 8 zu.

Die Figur 2 zeigt eine erfindungsgemäße Methode, um eine Faserdichtereduktion lokal zu erzeugen. Visualisiert ist ein Ausschnitt aus einem erfindungsgemäß gebildeten Hohlfaserpaket mit mehreren gewickelten oder gefalteten Lagen L1,...,L4.

Zwischen den Lagen L2 und L3 ist ein Platzhalter 13 angeordnet, der sich entweder über die gesamte axiale Länge der Hohlfasern erstrecken kann oder aber auch eine kleinere Länge aufweist als eine jeweilige Hohlfaser und der dann z.B. irgendwo zwischen den axialen Enden der Hohlfasern angeordnet werden kann, z.B. in einem axial endseitigen Bereich.

In dieser Ausführung hat der Platzhalter 13 einen Querschnitt, der größer ist als der Querschnitt jeder Faser 9. An gegenüberliegenden Bereichen 14 des Platzhalters 13 entstehen dort Freiräume, wo die Lagen L2 und L3 sich vom Platzhalter abheben und wieder zusammengeführt sind, welche die gewünschte Dichtereduktion bewirken. Der Platzhalter selbst kann auch durch sein eigenes Volumen zur Dichtereduktion beitragen, wenn er von Blut durchströmbar ist, wofür er als perforiertes Rohr ausgebildet sein kann. Der Platzhalter kann aber auch im Querschnitt massiv ausgebildet sein, so dass nur die Bereiche 14 dichtereduzierend wirken. Gemäß den vorherigen Ausführungen kann dieser Platzhalter 13 aber auch selbst eine stoffpermeable Hohlfaser bilden, die am Stoffaustausch teilnimmt.

Bezogen auf die Figur 1 können z.B. im Bereich 8, also oben und radial innen sowie im Bereich 7, also unten und radial außen solche Platzhalter angeordnet werden um dort lokal die Strömung zu begünstigen.

Sofern die Platzhalter axial endseitig im gewickelten / gefalteten Paket zugänglich sind, können diese vor einem Verpotten der Fasern aus dem Paket entfernt werden. Sofern Sie verbleiben, werden sie bevorzugt in die Verpottungsmasse mit eingeschlossen.

## Patentansprüche

1. Auf einen Kern gewickeltes Hohlfaserpaket aus einer Vielzahl stoffpermeabler Hohlfasern (9) von wenigstens einer Hohlfasermatte, in welcher die Hohlfasern mit Kettfäden auf Abstand gehalten sind, **dadurch gekennzeichnet, dass** im Hohlfaserpaket im Querschnitt senkrecht zur Hohlfasererstreckung die Dichte an Hohlfasern (9) lokal unterschiedlich ist, wobei ein Bereich geringerer Faserdichte erzeugt ist durch wenigstens einen in das Hohlfaserpaket, zumindest axial endseitig eingesetzten Platzhalter (13), der zwischen zwei Lagen von durch Kettfäden verbundenen Hohlfasern (9) eingewickelt ist.

2. Hohlfaserpaket nach Anspruch 1, **dadurch gekennzeichnet, dass** der Platzhalter (13) einen Querschnitt größer als eine Hohlfaser (9) aufweist.

3. Hohlfaserpaket nach Anspruch 1 oder 2, **dadurch gekennzeichnet, dass** ein Platzhalter selbst auch durch eine stoffpermeable Hohlfaser gebildet ist, insbesondere die einen Durchmesser aufweist, der größer ist als die überwiegende Anzahl der anderen stoffpermeablen Hohlfasern, wobei eine als Platzhalter ausgebildete Hohlfaser separat zu den Hohlfasern der Matte ist.

4. Vorrichtung für den Stoffaustausch zwischen Blut und einem Austauschmedium, insbesondere Gas/Gasgemisch, umfassend eine blutdurchströmbare Kammer (2), in der eine Vielzahl stoffpermeabler Hohlfasern (9) von wenigstens einer Hohlfasermatte, in welcher die Hohlfasern mit Kettfäden auf Abstand gehalten sind, als ein in mehreren Lagen auf einen Kern gewickeltes Hohlfaserpaket angeordnet ist, wobei die Hohlfasern (9) von dem Austauschmedium durchströmbar und von dem Blut umströmbar sind, **dadurch gekennzeichnet, dass** das Hohlfaserpaket nach einem der vorherigen Ansprüche 1 bis 3 ausgebildet ist.

5. Verfahren zur Herstellung eines Hohlfaserpakets einer Vorrichtung für den Stoffaustausch zwischen Blut und einem Austauschmedium, insbesondere einem Gas/Gasgemisch, bei dem wenigstens eine Matte mit einer Vielzahl stoffpermeabler Hohlfasern (9), die in der Matte durch Kettfäden zueinander beabstandet gehalten sind, zur Bildung des Hohlfaserpakets in mehreren Lagen auf einen Kern (3) gewickelt wird, **dadurch gekennzeichnet, dass** durch Beeinflussung des Abstandes der Hohlfasern (9) zueinander vor oder während des Wickelns im Querschnitt senkrecht zur Hohlfasererstreckung Bereiche (7, 8, 10, 11, 12, 14) mit unterschiedlicher Dichte an Hohlfasern (9) erzeugt werden, wobei die Beeinflussung des Abstandes der Hohlfasern (9) zueinander erfolgt durch Einwickeln von wenigstens einem Platzhalter (13) zwischen zwei Lagen (L) wenigstens einer Hohlfasermatte.

6. Verfahren nach Anspruch 5, **dadurch gekennzeichnet, dass** der wenigstens eine Platzhalter nur im Bereich der Faserenden eingewickelt wird.

## Claims

1. Hollow-fibre pack from a multiplicity of mass-permeable hollow fibres (9) of at least one hollow-fibre mat wound onto a core, in which hollow-fibre pack the hollow fibres are kept so as to be spaced apart by warp threads, **characterized in that** the density of hollow fibres (9) in the cross section perpendicular to the hollow-fibre extent in the hollow-fibre pack differs locally, wherein a region of lower fibre density is generated by at least one placeholder (13) which is inserted into the hollow-fibre pack at least axially at the end side and which is wrapped between two tiers of hollow fibres (9) that are connected by warp threads.

2. Hollow-fibre pack according to Claim 1, **characterized in that** the placeholder (13) has a cross section that is larger than a hollow fibre (9).

3. Hollow-fibre pack according to Claim 1 or 2, **characterized in that** a placeholder per se is also formed by a mass-permeable hollow fibre which in particular has a diameter that is larger than the predominant number of the other mass-permeable hollow fibres, wherein a hollow fibre that is configured as a placeholder is separate from the hollow fibres of the mat.

4. Device for the transfer of mass between blood and a transfer medium, in particular a gas/gas mixture, comprising a chamber (2) which can be passed through by a flow of blood and in which a multiplicity of mass-permeable hollow fibres (9) of at least one hollow-fibre mat in which the hollow fibres are kept so as to be spaced apart is disposed as a hollow-fibre pack which in a plurality of tiers is wound onto a core, wherein the hollow fibres (9) are able to be passed through by the transfer medium and be surrounded by a flow of blood, **characterized in that** the hollow-fibre pack is configured according to one of the preceding Claims 1 to 3,

5. Method for producing a hollow-fibre pack of a device for the transfer of mass between blood and a transfer medium, in particular a gas/gas mixture, in which method at least one mat having a multiplicity of mass-permeable hollow fibres (9) which by warp threads are kept so as to be mutually spaced apart in the mat is wound in a plurality of tiers onto a core (3) so as to form the hollow-fibre pack, **characterized in that**, in the cross section perpendicular to the extent of the hollow fibres, regions (7, 8, 10, 11, 12, 14) having dissimilar densities of hollow fibres (9) are generated by influencing the mutual spacing of the hollow fibres (9) before or during the winding, wherein the influencing of the mutual spacing of the hollow fibres (9) takes place by wrapping at least one placeholder (13) between two tiers (L) of at least one hollow-fibre mat.

6. Method according to Claim 5, **characterized in that** the at least one placeholder is wrapped only in the region of the fibre ends.

## Revendications

1. Paquet de fibres creuses enroulé sur un noyau composé d'une pluralité de fibres creuses (9) perméables à une substance d'au moins une natte de fibres creuses dans laquelle les fibres creuses sont maintenues écartées par des fils de chaîne, **caractérisé en ce que** dans le paquet de fibres creuses, en coupe transversale perpendiculairement par rapport à la projection des fibres creuses, la densité en fibres creuses (9) est localement différente, une zone à la densité de fibres plus faible étant produite par au moins un élément de substitution (13) introduit au moins axialement du côté de l'extrémité dans le paquet de fibres creuses, lequel est enroulé entre deux couches de fibres creuses (9) reliées par des fils de chaîne.

2. Paquet de fibres creuses selon la revendication 1, **caractérisé en ce que** l'élément de substitution (13) possède une section transversale plus grande qu'une fibre creuse (9).

3. Paquet de fibres creuses selon la revendication 1 ou 2, **caractérisé en ce qu'**un élément de substitution lui-même est également formé par une fibre creuse perméable à une substance, laquelle possède notamment un diamètre qui est supérieur à la grande majorité des autres fibres creuses perméables à une substance, une fibre creuse réalisée sous la forme d'un élément de substitution étant séparée des fibres creuses de la natte.

4. Procédé d'échange de substance entre le sang et un fluide d'échange, notamment un gaz/mélange gazeux, comprenant une chambre (2) à travers laquelle peut s'écouler le sang, dans laquelle sont disposées une pluralité de fibres creuses (9) perméables à une substance d'au moins une natte de fibres creuses, dans laquelle les fibres creuses sont maintenues écartées par des fils de chaîne, sous la forme d'un paquet de fibres creuses enroulé en plusieurs couches sur un noyau, les fibres creuses (9) pouvant être traversées par un flux du fluide d'échange et ne pouvant pas être traversées par un flux de sang, **caractérisé en ce que** le paquet de fibres creuses est configuré selon l'une des revendications 1 à 3 précédentes.

5. Procédé de fabrication d'un paquet de fibres creuses d'un dispositif pour l'échange de substance entre le sang et un fluide d'échange, notamment un gaz/mélange gazeux, avec lequel au moins une natte comprenant une pluralité de fibres creuses (9) perméables à une substance, qui sont maintenues espacées les unes des autres dans la natte par des fils de chaîne, est enroulée en plusieurs couches sur un noyau (3) en vue de former le paquet de fibres creuses, caractérisé en ce des zones (7, 8, 10, 11, 12, 14) ayant des densités de fibres creuses (9) différentes en coupe transversale perpendiculairement par rapport à la projection des fibres creuses sont produites en influençant l'écart des fibres creuses (9) entre elles avant ou pendant l'enroulement, l'influence de l'écart des fibres creuses (9) entre elles s'effectuant en emballant d'au moins un élément de substitution (13) entre deux couches (L) d'au moins une natte de fibres creuses.

6. Procédé selon la revendication 5, **caractérisé en ce que** l'au moins un élément de substitution est seulement emballé dans la zone des extrémités des fibres.
